(19) Europäisches Patentamt
European Patent Office
Office européen des brevets

(11) Veröffentlichungsnummer: **0 383 024 A2**

## (12) EUROPÄISCHE PATENTANMELDUNG

(21) Anmeldenummer: **90100739.3**

(22) Anmeldetag: **15.01.90**

(51) Int. Cl.5: **C07H 15/04, C11D 3/32, A61K 7/00**

(30) Priorität: **13.02.89 DE 3904247**

(43) Veröffentlichungstag der Anmeldung: **22.08.90 Patentblatt 90/34**

(84) Benannte Vertragsstaaten: **AT BE CH DE DK ES FR GB GR IT LI LU NL SE**

(71) Anmelder: **SÜDZUCKER AKTIENGESELLSCHAFT MANNHEIM/OCHSENFURT**
**Maximilianstrasse 10**
**D-6800 Mannheim 1(DE)**

(72) Erfinder: **Kunz, Markwart, Dr. rer. nat.**
**Dipl.-Chem.**
**Am Kötherberg 6**
**D-3300 Braunschweig(DE)**
Erfinder: **Schneider, Bernd, Dr. rer. nat.**
**Dipl.-Chem.**
**Mertesheimer Strasse 25 a**
**D-6719 Ebertsheim(DE)**

(74) Vertreter: **Körber, Wolfhart, Dr.rer.nat. et al**
**Patentanwälte Dipl.-Ing. H. Mitscherlich Dipl.-Ing. K. Gunschmann Dr.rer.nat. W. Körber Dipl.Ing. J. Schmidt-Evers Dipl.-Ing. W. Melzer Steinsdorfstrasse 10**
**D-8000 München 22(DE)**

## (54) N-Polyhydroxy-N'-alkylharnstoffderivate, Verfahren zu ihrer Herstellung und Verwendung derselben.

(57) Die Erfindung betrifft N-Polyhydroxy-N'-alkylharnstoffderivate entsprechend den Formeln

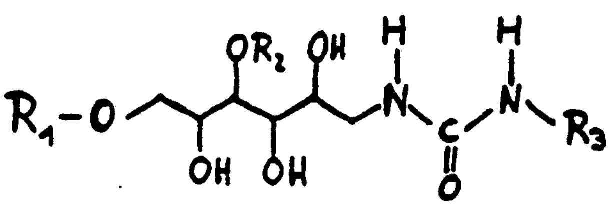

(I)

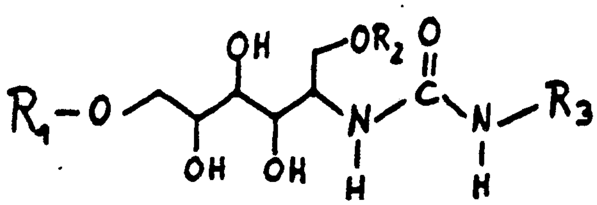

(II)

oder

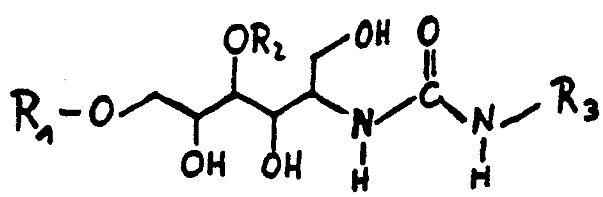

(III)

EP 0 383 024 A2

worin hedeuten: $R_1$ = H oder ein Mono- oder Oligosaccharid,
$R_2$ = H oder ein Mono- oder Oligosaccharid, sowie
$R_3$ = $C_nH_{2n+1}$ mit n = 4 bis 21

und Mischungen derselhen. Diese Harnstoffderivate sind amphiphil und finden daher Verwendung entsprechend ihren HLB-Werten unter anderem als nichtionogene Tenside, als Emulgatoren oder als Feuchthaltemittel in kosmetischen Formulierrnttgen. Ihre Herstellung erfolgt durch Umsatz von Aminopolyolen (erhalten zum Beispiel durch reduktive Aminierung von Sacchariden) mit Alkylisocyanaten mit 4 bis 21 C-Atomen im Alkylrest in Anwesenheit von Wasser und/oder Alkoholen oder von inerten aprotischen Lösungsmitteln.

# N-Polyhydroxy-N′-alkylharnstoffderivate, Verfahren zu ihrer Herstellung und Verwendung derselben

Die vorliegende Erfindung betrifft N-Polyhydroxy-N′-alkylharnstoffderivate entsprechend den im Anspruch 1 angeführten Formeln I, II oder III und deren Mischungen, die Herstellung derselben aus von Sacchariden abgeleiteten Aminopolyolen und Alkylisocyanaten mit 4 bis 21 C-Atomen im Alkylrest, sowie die Verwendung der Additionsprodukte als amphiphile Verbindungen. Amphiphile Harnstoffderivate, gebildet durch Reaktion von diesen Aminopolyolen mit den höheren Alkylisocyanaten, sind eine in der Literatur noch nicht beschriebene Verbindungsklasse. Die Konstitution der dabei erhaltenen und beanspruchten Verbindungen entsprechend den Formeln

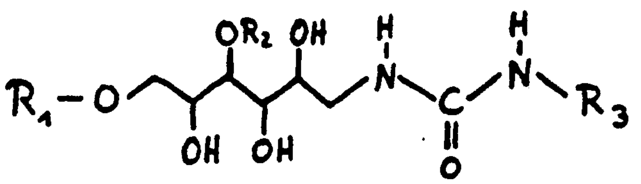 (I)

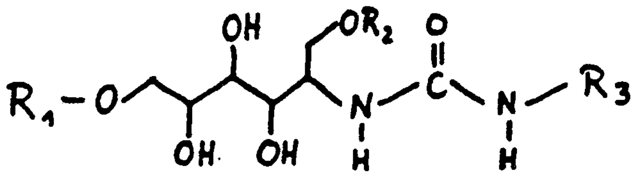 (II)

oder

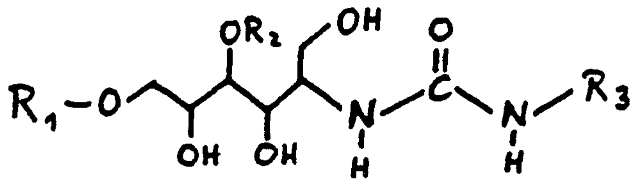 (III)

mit der im Anspruch 1 angeführten Definition der Substituenten $R_1$, $R_2$ und $R_3$ ist durch $^{13}C$-NMR-Spektren gesichert.

Die durch reduktive Aminierung aus reduzierenden Mono-oder Oligosacchariden erhältlichen Aminopolyole (vergleiche DE 35 38 451, EP 86114683.5, US 916,769 und DE 36 25 931, EP 87110615.9, US 080,266) reagieren in an sich bekannter Weise unter selektiver Addition mit Alkylisocyanaten mit 4 bis 21 C-Atomen im Alkylrest zu amphiphilen Harnstoffderivaten. Die Durchführung der Reaktion bei tiefen Temperaturen zwischen -10 bis 30C und in Gegenwart von Wasser und/oder Alkoholen oder von inerten aprotisch polaren Lösungsmitteln ergibt einen nahezu quantitativen Umsatz zu Verbindungen gemäß den Formeln I, II oder III. Die Isolierung der Produkte gestaltet sich in einfacher Weise durch Fällung in fester Form oder durch Verdampfen des Lösungsmittels.

Geeignete Aminopolyole sind zum Beispiel: Glucamin, Mannamin, Maltamin, Isomaltamin oder Trehalamin (erhalten durch reduktive Aminierung von Trehalulose = 1→1-Glucosylfructose) und deren Mischungen. Geeignete Alkylisocyanate sind zum Beispiel: Hexyl-, Octyl-, Dodecyl- und Palmitylisocyanat; von besonderem Vorteil ist Dodecylisocyanat.

Die Reaktivität sowohl des Isocyanats als auch des entsprechenden Nucleophils hängt bei dieser Additionsreaktion unter anderem von der Struktur des Alkylrestes am Isocyanat als auch von der Basizität des Nucleophils ab. Im Falle der hier eingesetzten Aminopolyole mit sowohl OH-Gruppen als auch $NH_2$-Gruppen reagiert die $NH_2$-Gruppe aufgrund ihrer größeren Basizität bevorzugt ab. Andererseits gilt für die Alkylisocyanate, daß in Abhängigkeit von der Anzahl der C-Atome im Alkylrest die Reaktivität verlangsamt wird; bei dem $C_{12}$-Isocyanat beispielsweise wurden Konkurrenzreaktionen mit Wasser oder Methanol bei Temperaturen zwischen 0 °C und 20 °C nicht beobachtet. Bei der anspruchsgemäßen Umsetzung wird das Alkylisocyanat in Mengen his zu 1 Mol eingesetzt; ein weiterer Zusatz hätte Nebenreaktionen zur Folge.

Eine Reinigung der amphiphilen Harnstoffderivate (zur Entfernung des gegebenenfalls nicht umgesetzten Aminopolyols oder anderer Reaktionsprodukte des Isocyanats) kann unter anderem durch Kationenaustauscherharze in wäßriger, wäßrig-alkoholischer oder alkoholischer Lösung erfolgen.

In der Literatur sind zwar schon N-substituierte Derivate von Aminodesoxyzuckern oder Aminocyclitolen,

welche zum Teil ebenfalls Harnstoffderivate sind, beschrieben, jedoch ist in diesen die N′-Alkylgruppe sehr kurzkettig bzw. liegt zum Beispiel zusätzlich eine Funktionalisierung durch Halogen- oder Nitrosogruppen vor. Verbindungen dieser Art werden bevorzugt als Antihiotika oder in der Tumorforschung verwendet.

Die in der vorliegenden Erfindung beschriebenen Harnstoffderivate mit höheren Alkylresten können dagegen entsprechend ihrem HLB-Wert als amphiphile Substanzen in weiten Bereichen Anwendung finden, zum Beispiel als nichtionogene Tenside, als Emulgatoren oder als Feuchthaltemittel in kosmetischen Formulierungen.

Beispiel 1

Umsetzung von Isomaltamin-2 mit Dodecylisocyanat zu N-Dodecyl-H′-2-desoxy-isomalt-harnstoff in wäßrig-methanolischer Lösung

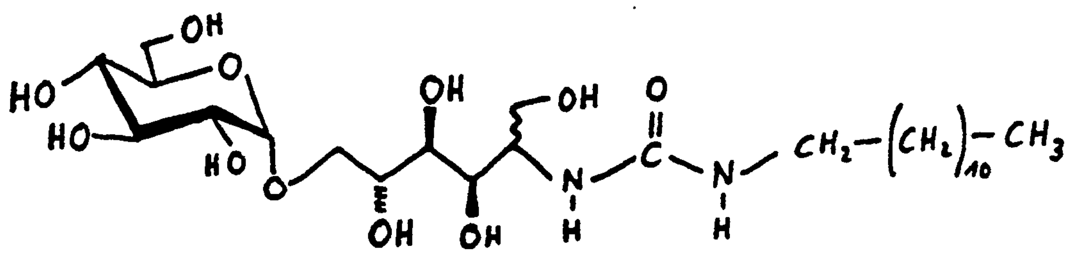

Eine Lösung von 50 g Isomaltamin-2 (0,15 mol) in 1,5 1 Methanol-Wassergemisch (2:1, v/v) wird unter Rühren bei 0° C tropfenweise mit 35,7 ml Dodecylisocyanat (0,15 mol) versetzt. Man rührt 1 h bei 0° C weiter und läßt das Reaktionsgemisch dann langsam sich auf Raumtemperatur erwärmen. Nach mehreren Stunden wird der Reaktionsansatz durch ausfallendes Produkt fest. Durch Erwärmen auf 60° C und langsames Abkühlen fällt das Produkt als flockiger weißer Niederschlag aus. Nach 1 d wird abgesaugt, mit Eiswasser gewaschen und bei 40°C im Vakuum getrocknet. Weiteres Produkt kann durch Einengen aus der Mutterlauge isoliert werden. Das Additionsprodukt ist das Gemisch zweier Isomerer.

Die Ausbeute betrug 65 g (80 %). Es wurde ein Schmelzpunkt von 184 - 186°C und eine Drehung von $[\alpha]_D^{20}$ 20 = + 54 (c = 1, Methanol) gefunden.

Das Additionsprodukt zeigte ein gutes Schaumtestverhalten (nach Ross und Miles) und war auch in Bezug auf Schleimhautverträglichkeit (Zein-Test, Draize-Test) voll einsetzbar.

Beispiel 2

Umsetzung eines Aminopolyolgemisches, welches durch reduktive Aminierung von Maltosesirup erhalten wurde, mit Dodecylisocyanat

50 g des Aminopolyolgemisches wird unter gleichen Bedingungen wie im Beispiel 1 mit Dodecylisocyanat umgesetzt. Die Reaktionslösung wird auf eine Kationenaustauschersäule ($H^+$-Form, zum Beispiel Amberlyst-15) aufgetragen und das Produkt durch Elution mit einem Methanol-Wassergemisch (2:1) von Aminspuren befreit. Das Eluat wird am Rotationsverdampfer bis zum verbleibenden gelb gefärbten pastösen Rückstand eingeengt.

**Ansprüche**

1. N-Polyhydroxy-N′alkylharnstoffderivate entsprechend den Formeln I, II oder III

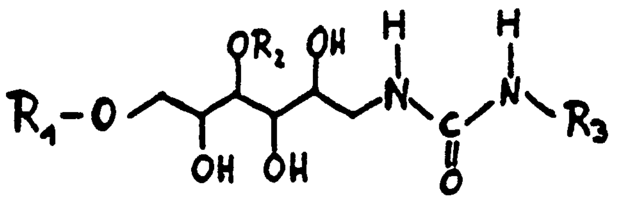

(I)

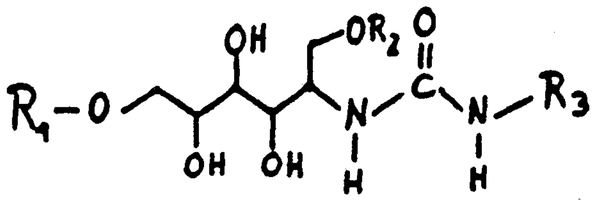

(II)

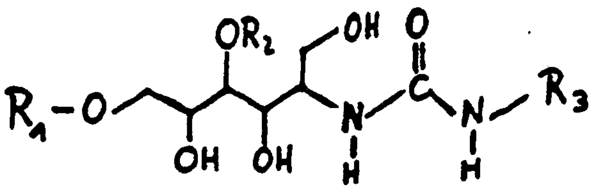

(III)

worin hedeuten: $R_1$ = H oder ein Mono- oder Oligosaccharid, $R_2$ = H oder ein Mono- oder Oligosaccharid, sowie $R_3$ = $C_nH_{2n+1}$ mit n = 4 bis 21, und Mischungen derselhen.

2. Verfahren zur Herstellung der Verbindungen gemäß Anspruch 1, dadurch gekennzeichnet, daß man in an sich bekannter Weise Aminopolyole mit Alkylisocyanaten mit 4 bis 21 C-Atomen im Alkylrest in wäßriger, wäßrig-alkoholischer oder alkoholischer Lösung bzw. Suspension oder in inerten aprotisch polaren Lösungsmitteln zur Reaktion bringt.

3. Verwendung der N-Polyhydroxy-N′-alkylharnstoffderivate nach Anspruch 1 entsprechend ihren HLB-Werten als amphiphile Substanzen für Reinigungszwecke im allgemeinen, für kosmetische Zwecke, auch als Hilfsstoffe zur Herstellung von kosmetischen Präparaten, bei der Lebensmittelherstellung, sowie für pharmazeutische Zwecke, auch als Hilfsstoffe zur Herstellung von pharmazeutischen Präparaten.